# EUROPÄISCHE PATENTSCHRIFT

(11) **EP 2 352 556 B1**
(45) Veröffentlichungstag und Bekanntmachung des Hinweises auf die Patenterteilung: **20.08.2014**
(21) Anmeldenummer: 09748234.3
(22) Anmeldetag: 17.10.2009
(51) Int. Cl.: A61N 5/10, G21K 5/04, G21K 5/10

(54) **BESTRAHLUNG VON ZUMINDEST ZWEI ZIELVOLUMEN**
IRRADIATION OF AT LEAST TWO TARGET VOLUMES
IRRADIATION D'AU MOINS DEUX VOLUMES CIBLES

(30) Priorität: 27.10.2008 DE 102008053321
(43) Veröffentlichungstag der Anmeldung: 10.08.2011
(73) Patentinhaber: GSI Helmholtzzentrum für Schwerionenforschung GmbH, 64291 Darmstadt (DE)
(72) Erfinder: BERT, Christoph, 63741 Aschaffenburg (DE); RIETZEL, Eike, 64331 Weiterstadt (DE)
(74) Vertreter: Mergel, Volker
(86) Internationale Anmeldenummer: PCT/EP2009/007461
(87) Internationale Veröffentlichungsnummer: WO 2010/049071

(56) Entgegenhaltungen:
- US-A1- 2004 254 448
- US-A1- 2007 286 343
- PEDRONI E ET AL: "THE 200-MEV PROTON THERAPY PROJECT AT THE PAUL SCHERRER INSTITUTE: CONCEPTUAL DESIGN AND PRACTICAL REALIZATION" MEDICAL PHYSICS, AIP, MELVILLE, NY, US, Bd. 22, Nr. 1, 1. Januar 1995 (1995-01-01) , Seiten 37-53, XP000505145 ISSN: 0094-2405

## Beschreibung

Die Offenbarung betrifft ein Verfahren zur Planung einer Bestrahlung mehrerer Zielvolumen mit einem Zielpunkte anfahrenden Strahl, ein Verfahren zur Bestrahlung solcher Zielvolumen, eine Vorrichtung zur Bestrahlung mehrerer Zielvolumen und ein Kontrollsystem zur Steuerung einer solchen Vorrichtung. Die Erfindung ist in den Ansprüchen definiert. Andere Ausführungsbeispiele sind nur beispielhaft. Die Bestrahlung eines Ziels mit einem verschiedene Punkte anfahrenden Strahl (Beam Scanning) ist an sich bekannt. So werden etwa bei der Bestrahlung von Tumoren beispielsweise Partikelstrahlen, vor allem Ionenstrahlen, insbesondere mit Protonen, α-Teilchen oder Kohlenstoffkernen, eingesetzt. Es werden Teile des Zielgebiets, die Zielpunkte, sequenziell nacheinander mit dem Strahl angefahren.

Partikelstrahlen sind für die Bestrahlung von Zielvolumen besonders vorteilhaft, da sie zu ihrem Ende hin ein Maximum in der Energiedeposition durchlaufen (Bragg-Peak). So können etwa auch eingebettete räumliche Strukturen effektiv bestrahlt werden, ohne die einbettende Umgebung allzu stark zu schädigen. Oft werden räumliche Zielgebiete schichtweise bestrahlt, wobei die die Eindringtiefe bestimmende Strahlenergie je Schicht konstant gewählt wird

(Isoenergieschicht). Bekannt ist auch das so genannte volumetrische Scanning (auch sog. "depth scanning"), bei dem die hintereinander angefahrenen Zielpunkte nicht notwendig einzelnen (Isoenergie-)Schichten zugeordnet sind. Die Erfindung betrifft grundsätzlich auch Ausführungsformen, bei denen der Strahl durch elektromagnetische Wellen gebildet wird.

Scanning-Verfahren ermöglichen eine an die Form des Ziels angepasste Bestrahlung durch Abtasten mit einem Strahl; es werden verschiedene Scanning-Verfahren unterschieden. Bewährt hat sich insbesondere das Raster-Scanning. Hier verweilt der Partikelstrahl an jedem von vielen anzufahrenden Rasterpunkten während einer vorbestimmten Zeitdauer oder deponiert an jedem dieser Rasterpunkte eine vorbestimmte Anzahl Partikel, wird aber zwischen den Rasterpunkten nicht oder nicht immer ausgeschaltet; die Erfindung ist jedoch grundsätzlich nicht auf Raster-Scanning eingeschränkt, sondern kann auch im Zusammenhang mit Spot-Scanning, einem kontinuierlichen oder diskontinuierlichen Scanning-Verfahren oder mit einem anderen Scanning-Verfahren Anwendung finden.

Ein Zielvolumen wird hier als ein Raumbereich verstanden, innerhalb dessen eine von einer Bedienperson, etwa von medizinischem Personal, festgelegte bzw. verschriebene Dosis deponiert werden soll.

Ein Zielpunkt ist ein etwa durch Angabe von drei kartesischen Raumkoordinaten (x, y, z) definierbarer Ort, der in der Regel innerhalb des zu bestrahlenden Objekts und insbesondere innerhalb des Zielvolumens liegt.

Wird Raster-Scanning eingesetzt ist zu beachten, dass die Rasterpunkte sich von den Zielpunkten unterscheiden können. I.d.R. fallen Zielpunkte nur auf einen Teil der Rasterpunkte; es werden also nicht sämtliche Rasterpunkte angefahren, da Raster üblicherweise nicht nur das oder die Zielvolumen überdecken, sondern auch die Umgebung, die nicht angefahren wird. Weiter können die Rasterpunkte in dem raumfesten Koordinatensystem angegeben werden, während die Zielpunkte ggf. in dem Koordinatensystem des Ziels betrachtet werden; insbesondere müssen Rasterpunkte und Zielpunkte nicht deckungsgleich übereinander liegen.

Es ist natürlich auch möglich, sowohl Rasterpunkte als auch Zielpunkte in einem gemeinsamen Koordinatensystem darzustellen; hier bietet es sich an, die Zielpunkte auf Rasterpunkte fallen zu lassen.

Im einfachsten Fall wird einem Zielvolumen und dessen Umgebung ein dieses Volumen überdeckendes Raster zugewiesenen, wobei die Rasterpunkte innerhalb des Zielvolumens Zielpunkten entsprechen und diesem Zielvolumen zugewiesen werden.

Ein Partikelstrahl ist ein Strahl mit einem definierten Querschnitt aus Partikeln und mit einem definierten, in der Regel schmalen, Spektrum der Partikelenergie. Die Partikelenergie ist die Energie des einzelnen Partikels beim Eintritt in das zu bestrahlende Objekt.

Wenn hier auf einen Partikelstrahl Bezug genommen wird, der auf einen Zielpunkt gerichtet ist, bedeutet dies, dass der Partikelstrahl (beispielsweise durch Ablenkmagnete) in x- und y-Richtung so gelenkt wird, dass der Zielpunkt beispielsweise im Schwerpunkt oder auf einer Linie (oder deren Verlängerung) maximaler Fluenz oder Dosis liegt, und dass der Zielpunkt im Bragg-Peak des Partikelstrahls liegt; es wird insbesondere vom Anfahren eines Zielpunktes gesprochen.

Bei der Planung einer Bestrahlung werden in der Regel pro Zielpunkt bzw. pro Rasterpunkt insbesondere folgende Größen festgelegt: laterale Position, Energie - bestimmend die Eindringtiefe, Fokus und Teilchenzahl.

Üblicherweise soll, etwa bei der Bestrahlung eines Tumors, eine bestimmte Verteilung der Dosis, also eine Zieldosisverteilung, insbesondere eine biologisch effektive Zieldosisverteilung für bestimmte Ionen, über das Zielvolumen erzielt werden. Die Zieldosisverteilung wird etwa als deponierte Energie pro Volumeneinheit quantifiziert. Verbreitet ist eine Angabe einer Dosis in Joule pro Kilogramm (Gray).

Grundsätzlich ist es bekannt, mehrere Zielvolumen während einer Sitzung zu bestrahlen. Bei der Bestrahlung einer Person können dies beispielsweise Lungentumore und Lymphknoten im Mediastinum sein. Die einzelnen Zielvolumen können dabei etwa mit der gleichen oder aber auch mit einer unterschiedlichen Dosis belegt werden.

Der Erfindung liegt die Aufgabe zu Grunde, ein vorteilhaftes Verfahren zur Planung einer Bestrahlung mehrerer Zielvolumen mit einem Zielpunkte anfahrenden Strahl, ein entsprechendes vorteilhaftes Verfahren zu Bestrahlung solcher Volumen, eine entsprechende vorteilhafte Vorrichtung zur Bestrahlung mehrerer Zielvolumen und ein vorteilhaftes Kontrollsystem zur Kontrolle einer solchen Vorrichtung anzugeben.

Die Aufgabe wird gelöst durch ein Verfahren zur Planung einer Bestrahlung von zwei, oder auch mehr als zwei, Zielvolumen mit einem Zielpunkte anfahrenden Strahl zur Dosisdeposition, kurz Deposition, einer ersten Zieldosisverteilung in einem ersten der zwei Zielvolumen und einer zweiten Zieldosisverteilung in einem zweiten der zwei Zielvolumen. Das Verfahren ist gekennzeichnet durch die Schritte: Zuordnen von Zielpunkten zu einem der Zielvolumen, Feststellen einer Überlagerung von einer ersten Deposition verursacht durch das Anfahren eines dem ersten Zielvolumen zugeordneten Zielpunkts mit einer zweiten Deposition verursacht durch das Anfahren eines dem zweitem Zielvolumen zugeordneten Zielpunkts und Anpassen der Planung für zumindest einen der Zielpunkte, dessen Anfahren zu der Überlagerung der ersten und zweiten Deposition beiträgt.

Bevorzugte Ausgestaltungen der Erfindung sind in abhängigen Ansprüchen angegeben und werden im Folgenden näher erläutert.

Ein Verfahren zur "Planung einer Bestrahlung" braucht nicht selbst eine Bestrahlung zu umfassen, sondern kann auch unabhängig von einer Bestrahlung durchgeführt werden; insbesondere kann die Planung einer Bestrahlung vorangehen. Der sprachlichen Einfachheit halber wird dies in der Beschreibung nicht immer ausdifferenziert. Die vorangehende und die folgende Beschreibung der einzelnen Verfahrensmerkmale bezieht sich sowohl auf das Planungsverfahren als auch auf das Bestrahlungsverfahren, ohne dass dies in jedem Fall explizit erwähnt ist.

Die Erfindung beruht auf der Erkenntnis, dass bei der Bestrahlung von mehreren Zielvolumen nach jeweils selbstständig geplanten Zieldosisverteilungen Überlagerungen der Deposition auftreten können; solche Überlagerungen können dazu führen, dass die tatsächliche Deposition lokal deutlich größer ist als es der jeweiligen Zieldosisverteilung entspricht.

Wird beispielsweise ein innerhalb eines Körpers liegendes erstes Zielvolumen entsprechend einer ersten Zieldosisverteilung bestrahlt, so wird auch eine gewisse Dosis entlang des Einstrahlungskanals außerhalb des ersten Zielvolumens deponiert. Befindet sich ein zweites Zielvolumen mit einer eigenen selbstständig bestimmten zweiten Zieldosisverteilung innerhalb des Einstrahlungskanals des ersten Zielvolumens, so wird die gesamte deponierte Dosis innerhalb des zweiten Zielvolumens größer als nach der zweiten Zieldosisverteilung sein.

Eine solche Überlagerung kann etwa auch durch einen Überlapp von Zielvolumen, oder auch, wenn sie nahe beieinander liegen, entstehen. Dies kann etwa bei einem Überlapp bestimmter, beispielsweise anatomischer, Strukturen der Fall sein.

Es ist auch bekannt, Zielvolumen mit einem ebenfalls zu bestrahlenden Sicherheitssaum zu versehen, was, wegen der größeren Ausdehnung, Überlagerungen begünstigt. Durch solche Sicherheitssäume werden etwa Bewegungen der zu bestrahlenden Strukturen bzw. Fehlpositionierungen berücksichtigt. Ein Zielvolumen mit Sicherheitssaum wird auch "Planungszielvolumen" genannt.

Weiter wird beim Anfahren eines Zielpunkts die Dosis nicht punktförmig deponiert, sondern erfasst auch eine Umgebung des Zielpunkts. Auch daher ist es möglich, dass ein Zielpunkt, dessen nominale Position nicht in einer Überlagerung liegt, dennoch zu einer Überlagerung beiträgt.

Überlagerungen werden insbesondere auch durch sich bewegende Zielvolumen begünstigt. Wenn sich beispielsweise bei der Bestrahlung einer Person ein erstes Zielvolumen um 1.0 cm Zentimeter cranial bewegt und ein zweites angrenzendes bzw. nahe gelegenes oder überlappendes Zielvolumen jedoch nur um 0.5 cm, kann dies zu einer Überlagerung von Depositionen oder zu einer Veränderung einer solchen Überlagerung führen, welche in der Regel nicht durch eine einfache Anpassung der Position und/oder der Orientierung der zu bestrahlenden Person kompensiert werden kann.

Auch durch ein Nachführen einzelner Rasterpunkte, s.u., kann, insb. bei unterschiedlicher Zieldosisverteilung der Zielvolumen, die interne (Relativ -)Bewegung ggf. nicht ausgeglichen werden. Beim Nachführen können sich etwa die zu bestrahlenden Volumina relativ zueinander verschieben. Dann ist es vorteilhaft für die "veränderten" Überlappbereiche bspw. jeweils die größere erforderliche Dosis zu wählen. Dies kann beim Nachführen zu "neuen" Gebieten führen, die zuvor nicht als Überlappbereich aufgetreten sind.

Weiter basiert die Erfindung auch auf der Erfahrung, dass eine Bestrahlung typischerweise in mehreren Durchgängen, etwa Fraktionen bei fraktionierter Bestrahlung, stattfindet, wobei in jedem Durchgang nur ein Bruchteil der gesamten zu deponierenden Dosis deponiert wird. Der zeitliche Abstand zwischen Fraktionen beträgt in der Regel einen oder mehrere Tage. Die gesamte Bestrahlung erstreckt sich deshalb in der Regel über mehrere Wochen. Innerhalb dieses Zeitraums können die Zielvolumen ihre Position, Größe und Gestalt verändern; was unerwünschte Überlagerungen ebenfalls begünstigt.

Die Erfindung basiert auf der Idee, die Zielpunkte bestimmten Zielvolumen zuzuordnen und Überlagerungen von Depositionen festzustellen, die durch das Anfahren von Zielpunkten mit unterschiedlichen zugeordneten Zielvolumen verursacht werden, um dann mit diesem Wissen die Bestrahlung für zumindest einen der Zielpunkte, dessen Anfahren zu der Überlagerung beiträgt, anzupassen.

Nach der Erfindung wird also ein Teil der Zielpunkte, idealerweise werden sämtliche Zielpunkte, jeweils einem der Zielvolumen zugeordnet. In einem besonders einfachen Fall wird ein erster Teil der Zielpunkte einem ersten Zielvolumen zugeordnet und ein zweiter Teil der Zielpunkte einem zweiten Zielvolumen. Eine Zuweisung von einzelnen Zielpunkten zu mehreren Zielvolumen ist nach der Erfindung nicht ausgeschlossen.

Der Begriff "Zuordnen" umfasst insbesondere Ausdrücke, die teilweise eine engere Bedeutung haben, wie etwa "Klassifizieren", mit einem "Label" versehen und "Kennzeichnen".

Vorzugsweise werden die Zielpunkte innerhalb eines Zielvolumens diesem Zielvolumen zugeordnet. Handelt es sich um ein eingebettetes Zielvolumen, so kann es sinnvoll sein, auch Punkte außerhalb des Zielvolumens, jedoch mit einer Deposition, etwa im Einstrahlungskanal, diesem Volumen zuzuordnen; der sprachlichen Einfachheit halber kann man auch diese Punkte als Zielpunkte bezeichnen.

Ist ein Raster gegeben, kann es sein, dass mehrere Zielpunkte auf einen Rasterpunkt fallen. So z.B., wenn sich ein erstes und ein zweites Zielvolumen überlappen; die Rasterpunkte innerhalb des Überlapps können dann sowohl als Zielpunkt nach der ersten Zieldosisverteilung als auch nach der zweiten Zieldosisverteilung ausgewählt sein.

In einer auf Rasterpunkten basierenden Darstellung kann man insofern von einer "Rasterpunktzieldosis" sprechen; gemeint ist hier die Zieldosis pro Rasterpunkt, die sich durch Addition der Zieldosen für die auf diesen Rasterpunkt fallenden Zielpunkte ergibt. In diesem Fall wird nach der Erfindung die Rasterpunktzieldosis angepasst. Weiter kann man in dieser Darstellung insbesondere auch davon sprechen, dass ein Rasterpunkt mehreren Zielvolumen zugeordnet werden kann, etwa insofern, als dass die auf diesen Rasterpunkt fallenden Zielpunkte jeweils unterschiedlichen Zielvolumen zugeordnet sind.

Im Extremfall kann sich auf diese Weise durch das Anfahren eines einzigen Rasterpunkts bereits eine Überlagerung ergeben, beispielsweise etwa bei zwei auf den Rasterpunkt fallenden Zielpunkten, welche unterschiedlichen Zielvolumen zugeordnet sind, durch das Anfahren des Rasterpunkts nach einer entsprechenden ersten Zieldosisverteilung und einer entsprechenden zweiten Zieldosisverteilung.

Zur Deposition der gesamten Rasterpunktzieldosis braucht der Rasterpunkt nicht für jeden auf den Rasterpunkt fallenden Zielpunkt getrennt angefahren werden; dies kann kumulativ geschehen.

Die tatsächlich an verschiedenen Zielpunkten deponierte Dosis wird bestimmt (s.u.) und vorzugsweise gespeichert und etwa in einer Tabelle abgelegt. Basierend auf solchen Daten kann die folgende Bestrahlung angepasst werden; eine Vordosis also auch während der Bestrahlung berücksichtigt werden. Für komplexere Fälle kann es dabei notwendig sein, für jeden bestrahlten Punkt die Dosisbeiträge auch an anderen Punkten - wie erwähnt sogar außerhalb des Zielvolumens - zu berücksichtigen, wobei unter einem Punkt grds. ein Volumenelement, auch Voxel genannt, verstanden werden kann.

So ist es möglich, durch Vergleich mit den Zieldosisverteilungen, Überlagerungen in dem oben dargestellten Sinn festzustellen. Anpassungen können insbesondere auch während der Bestrahlung, etwa von Durchgang zu Durchgang, vorgenommen werden. Bei der Planung einer Bestrahlung können Überlagerungen auch im Vorfeld anhand von Simulationen festgestellt werden.

Die Anpassung sollte idealerweise so erfolgen, dass insgesamt die tatsächlich deponierte Dosis den Zieldosisverteilungen möglichst nahe kommt. Dabei entspricht ein "Anpassen" nach der Erfindung zumindest einem Anpassen der Intensität der Bestrahlung, bspw. durch Änderung der Teilchenzahl pro Deposition oder durch Änderung von Fluss und Depositionszeit.

Überlappen sich beispielsweise zwei Zielvolumen, ist es etwa bei der Bestrahlung von Tumoren vorzuziehen, wenn im Überlapp die tatsächlich deponierte Dosis der höheren Zieldosis entspricht. Die Anpassung kann etwa dadurch erfolgen, dass ein Teil der Zielpunkte, die in dem Überlapp liegen nicht angefahren werden; man kann auch von "Inaktivieren" sprechen; bzw. von "Herausnahme aus dem Bestrahlungsplan". In diesem Fall können einfach die Zielpunkte für die geringere Zieldosisverteilung inaktiviert werden und die Zielpunkte für die höhere Zieldosisverteilung aktiv bleiben. Entsprechende Rasterpunkte werden hier im Überlapp dann nur noch entsprechend der höheren Zieldosisverteilung angefahren.

In einfachen Fällen beschränkt sich das Anpassen auf ein solches aktiv- bzw. inaktiv Schalten von Ziel- bzw. Rasterpunkten. Durch geeignete Kombinatorik von aktiv/inaktiv geschalteten Rasterpunkten kann dann angestrebt werden, die einzelnen Zielvolumen jeweils mit der erforderlichen Dosis zu belegen. Ein Anpassen kann insbesondere aber auch durch eine Verringerung der deponierten Dosis für entsprechende Ziel-/Rasterpunkte durchgeführt werden.

Wird keine Überlagerung festgestellt, ist keine Anpassung im Sinne der Erfindung erforderlich; alle Ziel- bzw. Rasterpunkte können in der Bestrahlungsplanung verbleiben.

Neben der Bestrahlung von Personen oder Tieren ist auch die Bestrahlung von organischem Material überhaupt, insbesondere von Zellen, oder auch die Bestrahlung anorganischer Materialien, etwa von Kunststoffen, relevant; etwa im Rahmen der Materialforschung.

Insgesamt betrachtet erlaubt es die Erfindung, die Zieldosisverteilungen für die einzelnen Zielvolumen unabhängig voneinander zu planen. Eventuelle Überlagerungen, insbesondere Überlagerungen die erst durch eine Bewegung der zu bestrahlenden Strukturen entstehen, können dann nach der Erfindung ggf. auch bei der Bestrahlung bzw. sogar schon vor der Bestrahlung berücksichtigt werden.

Vorzugsweise geschieht das Anfahren der Zielpunkte im Rahmen von Raster-Scanning. Werden die Zielpunkte und die Rasterpunkte in einem gemeinsamen Koordinatensystem beschrieben, so fallen die Zielpunkte auf zumindest einen Teil der Rasterpunkte. Jedem einzelnen Zielvolumen kann ein eigenes Raster zugeordnet sein, es ist aber auch möglich sämtliche Zielvolumen in ein Raster einzubetten.

Bei einer bevorzugten Ausführungsform der Erfindung, wird die Bestrahlung in mehreren, zumindest in zwei, Durchgängen durchgeführt. Hier wird die insgesamt zu applizierende Dosis so über die einzelnen Durchgänge verteilt, dass sich insgesamt zumindest näherungsweise die Zieldosisverteilungen ergeben.

Eine Bestrahlung in mehreren Durchgängen ist grundsätzlich vorteilhaft, insbesondere da durch statistische Effekte Fehler ausgemittelt werden können.

Wie erwähnt ist es möglich, dass die zu bestrahlenden Strukturen ihre Position von Durchgang zu Durchgang relativ zueinander verändern. Die Lageänderung der Strukturen kann erfasst werden (s.u.) und die Zielvolumen entsprechend nachgeführt (s.u.) werden. Auf diese Weise können bedingt durch die Bewegung der Strukturen Überlagerungen von einem Durchgang zum nächsten Durchgang entstehen. Es ist besonders vorteilhaft, wenn vor jedem Durchgang gegebenenfalls aufgetretene Überlagerungen festgestellt werden, um die Bestrahlung erfindungsgemäß anzupassen.

Je nach zu bestrahlender Struktur kann eine Relativbewegung auf verschiedenen Zeitskalen stattfinden. Wird etwa eine Person bestrahlt, so hat man Bewegungen auf der Skala von Sekundenbruchteilen (Herzschlag), von Sekunden (Atmung), und Minuten bis Tagen (Verschiebungen und Gestaltänderungen von anatomischen Strukturen, insb. von inneren Organen).

Auch die Abstände zwischen den Durchgängen können recht unterschiedlich gestaltet sein, so kann ein Durchgang bei einer fraktionierten Bestrahlung einer Sitzung entsprechen, welche ggf. mehrere Tage auseinander liegen können. Es kann jedoch auch ein Durchgang pro Tag bzw. auch mehrere Durchgänge pro Tag durchgeführt werden. Etwa bei speziellen Mehrfachbestrahlungen sind die Durchgänge sogar auf nur einige Sekunden bzw. wenige Minuten verteilt.

Die zielvolumen werden mittels Röntgentomographie, Ultraschalldiagnostik bzw. Sonographie, optischer Kohärenztomographie, Magnetresonanztomographie bzw. Kernspintomographie, Computertomographie, Positronen-Emissions-Tomographie, Single-Photon-Emission-Computered-Tomographie (SPECT), elektromagnetischer Impedanz-Tomographie (EMIT), Neutronentomographie oder anderer Verfahren erfasst, die zur dreidimensionalen Abbildung, oder auch 2D oder 4D Abbildung, eines zu bestrahlenden Körpers oder von Teilen desselben geeignet sind. Dabei werden Veränderungen von Position, Größe und Form gegenüber der Situation, die dem ursprünglichen Bestrahlungsplan zugrunde lag, oder gegenüber der Situation bei vorangehenden Durchgängen erfasst.

Vorzugsweise wird nach jedem Durchgang die in dem Durchgang applizierte Dosis bestimmt bzw. berechnet. So kann der Verlauf der Bestrahlung gut mitverfolgt werden. Insbesondere ist es auch bevorzugt, nach jedem Durchgang der Bestrahlung die Summe aller bisher applizierten Dosen zu berechnen. Bei Abweichungen kann so eine neue Optimierung des Bestrahlungsplans für weitere Durchgänge unter Berücksichtigung der bereits applizierten Dosis durchgeführt werden.

Gegebenenfalls können bei einer solchen "Nachberechnung" auch systematische Änderungen erkannt werden, auf die mit einer auch neuen Bestrahlungsplanung reagiert werden kann

Wenn keine Veränderungen in dem zu bestrahlenden Objekt stattfänden, könnten die Datensätze für alle Durchgänge gleich gewählt werden. Zwischen den Durchgängen stattfindende Veränderungen motivieren Unterschiede zwischen den Datensätzen der einzelnen Durchgänge. Die beschriebene Änderung der Bestrahlungsplanung bzw. Bestrahlung kann als Adaptierung an die Veränderungen im zu bestrahlenden Objekt bezeichnet werden. Im Laufe einer Wiederholung des beschriebenen Verfahrens vor ausgewählten oder allen Durchgängen können systematische Änderungen erkannt werden, die darauf hindeuten, dass anstelle einer wiederholten Adaptierung des Bestrahlungsplans bzw. des Datensatzes eine vollständige neue Erstellung bzw. neue Optimierung des Bestrahlungsplans vorteilhaft wäre

Ändert sich die Lage und/oder die Form zumindest eines der Zielvolumen, so kann sich die Überlagerung ändern, insbesondere, wenn sich die Lage der Zielvolumen relativ zueinander ändert.

Besteht bereits ein Bestrahlungsplan, so ist es bevorzugt, diesen aufgrund der Lage und/oder Formänderung zumindest eines der Zielvolumen zu ändern, anstatt einen neuen Bestrahlungsplan zu erstellen.

Insbesondere werden dabei nach geänderter Planung diejenigen Zielpunkte mit einer geänderten Dosis belegt, welche sonst durch Überlagerung eine fehlerhaften Dosis begründen; ggf. ist dies nur ein kleiner Teil aller Zielpunkte. Dies betrifft vor allem die Zielpunkte in einem Sicherheitssaum. Im einfachsten Fall werden Zielpunkte nicht mehr oder aber sogar neu angefahren.

Dies kann insbesondere dadurch implementiert werden, dass verschiedene räumliche Lagen und/oder Formen zumindest eines der Zielvolumina von vornherein berücksichtigt werden. Insbesondere können so für Zielpunkte, die im Sicherheitssaum liegen, verschiedene Dosisbeiträge, abhängig von den verschiedenen räumlichen Lagen und/oder Formen zumindest eines der Zielvolumina, berechnet werden. Dies ist vorteilhaft, da gerade bei diesen Zielpunkten oftmals eine fehlerhafte Dosis durch eine Lage- bzw. Formveränderung zumindest eines der Zielvolumina auftritt. Wenn dann tatsächlich eine Lageänderung bzw. Formveränderung auftritt, können die vorberechneten Dosisbeiträge zur Änderung eines bereits bestehenden Bestrahlungsplans verwendet werden. Auf diese Weise kann auf die Lage- bzw. Formveränderung schnell reagiert werden und ein Bestrahlungsplan schnell angepasst werden.

Wie oben bereits erwähnt, kann es auch notwendig sein, Dosisbeiträge von Ziel-/Rasterpunkten auch an entfernt liegenden Voxeln abzuspeichern, auch außerhalb der entsprechenden Zielvolumen.

Vorteilhafterweise werden für jedes Zielvolumen und jeden jeweils zugehörigen Zielpunkt auch solche Dosisbeiträge in der Planung bereits berücksichtigt, so dass bei der Adaption von Durchgang zu Durchgang, etwa täglich, schnell auf die Dosisbeiträge zurückgegriffen werden kann. Damit lässt sich dann die gewünschte Dosisverteilung wiederum etwa über geeignete Kombination insbesondere anzufahrender Rasterpunkte für jedes Zielvolumen anpassen.

Wie bereits erwähnt ist es i.d.R. sinnvoll, die Zielvolumen bzw. die entsprechenden Rasterpunkte den zu bestrahlenden Strukturen nachzuführen; hier wird ebenfalls von einem Anpassen gesprochen, wobei sich der Begriff Anpassen hier insb. auf die Position der Punkte bezieht. Ein solches Nachführen ist etwas detaillierter unten und ausführlich in der DE 10 2006 044 139 A1 beschrieben.

Bei einem solchen Nachführen, hier beispielsweise bei der Bestrahlung eines Tumors unter Einsatz von Raster-Scanning, werden etwa die x- und y-Positionen der Rasterpunkte sowie die erforderliche Energie für die aktuelle Anatomie festgelegt. Dabei werden zunächst die Volumen zueinander registriert und die bestimmten Transformationsparameter auf die Rasterpunktpositionen in der Anatomie angewendet, wobei die erforderliche Energie anhand der aktuellen Anatomie ggf. neu zu bestimmen ist. Ggf. ist auch eine Interpolation auf ein wiederum reguläres Raster notwendig, sowie ggf. Anpassungen der Teilchenzahl pro Rasterpunkt. Transformationen können entweder für die Anatomie als ganzes oder aber auch für die einzelnen Zielvolumen erfolgen.

Vorzugsweise ist zumindest eines der Zielvolumen, idealerweise sind sämtliche Zielvolumen jeweils, mit einem ebenfalls zu bestrahlenden Sicherheitssaum versehen. Die Bestrahlung wird so, wie erwähnt, robuster gegenüber Fehlern, etwa in der Positionierung der Zielvolumen. Überlagerungen sind in diesem Fall besonders wahrscheinlich, da die Zielvolumen entsprechend vergrößert sind. Umso relevanter sind ein Feststellen von Überlagerungen und eine entsprechende Anpassung der Bestrahlung.

Es ist bevorzugt, Punkten des zu bestrahlenden Objekts Information zuzuordnen, die über das Zuordnen von Zielpunkten zu Volumen hinausgeht bzw. auch unabhängig von einer solchen Zuordnung sein kann. Etwa für einen bestimmten Punkt beispielsweise die Information, dass hier zwar durch das Anfahren eines Zielvolumens Energie deponiert ist, jedoch der Punkt nicht innerhalb dieses Zielvolumens liegt. Das kann etwa für einen Punkt im Einstrahlungskanal der Fall sein. Insbesondere kann Zielpunkten weitere Information, über eine Zuordnung zu einem Zielvolumen hinaus, zugeordnet werden. Etwa die bisher deponierte Dosis. Die entsprechenden Daten können zur weiteren Nutzung etwa in einer Tabelle abgelegt werden.

Die erfindungsgemäße Aufgabe wird ebenfalls gelöst durch ein Verfahren zur Bestrahlung von zwei Zielvolumen mit einem Zielpunkte anfahrenden Strahl zur Deposition einer ersten Zieldosisverteilung in einem ersten der zwei Zielvolumen und einer zweiten Zieldosisverteilung in einem zweiten der zwei Zielvolumen. Das Verfahren ist gekennzeichnet durch die Schritte: Zuordnen von Zielpunkten zu einem der Zielvolumen, Feststellen einer Überlagerung von einer ersten Deposition verursacht durch das Anfahren eines dem ersten Zielvolumen zugeordneten Zielpunkts mit einer zweiten Deposition verursacht durch das Anfahren eines dem zweitem Zielvolumen zugeordneten Zielpunkts und Anpassen der Bestrahlung für zumindest einen der Zielpunkte, dessen Anfahren zu der Überlagerung der ersten und zweiten Deposition beiträgt.

Vorzugsweise umfasst das Verfahren zur Bestrahlung das oben dargestellte Verfahren zur Planung einer Bestrahlung; insbesondere auch in einer der bevorzugten Ausgestaltungen des Planungsverfahrens.

Die Aufgabe wird auch gelöst durch eine Vorrichtung zur Bestrahlung von zwei, oder auch mehr als zwei, Zielvolumen mit einem Zielpunkte anfahrenden Strahl zur Deposition einer ersten Zieldosisverteilung in einem ersten der zwei Zielvolumen und einer zweiten Zieldosisverteilung in einem zweiten der zwei Zielvolumen mit einer Strahlenquelle und einem Kontrollsystem zur Steuerung der Vorrichtung. Die Vorrichtung ist dadurch gekennzeichnet, dass das Kontrollsystem dazu ausgelegt ist, Zielpunkte zu einem der Zielvolumen zuzuordnen, eine Überlagerung von einer ersten Deposition verursacht durch das Anfahren eines dem ersten Zielvolumen zugeordneten Zielpunkts mit einer zweiten Deposition verursacht durch das Anfahren eines dem zweiten Zielvolumen zugeordneten Zielpunkts festzustellen und die Bestrahlung für zumindest einen der Zielpunkte, dessen Anfahren zu der Überlagerung der ersten und zweiten Deposition beiträgt, anzupassen.

So eine Bestrahlungsvorrichtung umfasst eine Strahlenquelle, insbesondere zur Erzeugung eines Partikelstrahls, insbesondere eines Ionenstrahls. Als Strahlenquelle kommt etwa ein Beschleuniger, insbesondere ein Synchrotron oder ein Cyclotron in Betracht.

Wird mit einem Ionenstrahl bestrahlt, so umfasst die Bestrahlungsvorrichtung vorzugsweise auch eine Scanning-Einrichtung, kurz einen Scanner, umfassend Scanning-Magnete zum Ablenken des Ionenstrahls. Weiter kann sie vorzugsweise ein System zur Energiemodulation, etwa für "volumetrisches Scanning" und "wobbling", umfassen.

Vorzugsweise ist die Vorrichtung zur Bestrahlung zur Ausführung eines der oben dargestellten Verfahren ausgelegt; insbesondere auch in einer der bevorzugten Ausgestaltungen der Verfahren.

Ebenfalls gelöst wird die Aufgabe durch ein Kontrollsystem zur Steuerung einer Vorrichtung zur Bestrahlung von zwei Zielvolumen mit einem Zielpunkte anfahrenden Strahl zur Deposition einer ersten Zieldosisverteilung in einem ersten der zwei Zielvolumen und einer zweiten Zieldosisverteilung in einem zweiten der zwei Zielvolumen. Das Kontrollsystem ist dadurch gekennzeichnet, dass es dazu ausgelegt ist, Zielpunkte zu einem der Zielvolumen zuzuordnen, eine Überlagerung von einer ersten Deposition verursacht durch das Anfahren eines dem ersten Zielvolumen zugeordneten Zielpunkts mit einer zweiten Deposition verursacht durch das Anfahren eines dem zweiten Zielvolumen zugeordneten Zielpunkts festzustellen und die Bestrahlung für zumindest einen der Zielpunkte, dessen Anfahren zu der Überlagerung der ersten und zweiten Deposition beiträgt, anzupassen.

Im Unterschied zu der Bestrahlungsvorrichtung umfasst das Kontrollsystem selbst keine Strahlenquelle. Wird das Kontrollsystem zur Steuerung einer Bestrahlungsvorrichtung zur Bestrahlung von Menschen oder Tieren eingesetzt, so spricht man auch von einem Therapiekontrollsystem (TCS). Vorzugsweise verfügt das Kontrollsystem über zumindest eine Einrichtung zum Feststellen der Parameter der Bestrahlungsvorrichtung, wie etwa die Scannereinstellung und Strahleigenschaften, und/oder über eine Einrichtung zum Erfassen der zu bestrahlenden Strukturen.

Das Kontrollsystem kann etwa mit Hilfe eines Rechners oder eines Rechnersystems implementiert werden. So kann in einem solchen Rechner etwa Information betreffend die Energieaufteilung der Durchgänge und der isoenergetischen Schichten, die Zielpunkte, die Rasterpunkte, die Zieldosis pro Rasterpunkt, den Behandlungsplan und Kriterien für ein Beenden der Bestrahlung abgelegt sein. Ein Kriterium für das Beenden der Bestrahlung kann etwa das Erreichen der im Behandlungsplan festgelegten Zieldosis pro Zielpunkt sein. Es bietet sich an, die Zieldosis pro Zielpunkt in einer Tabelle abzulegen.

Vorzugsweise wird das Kontrollsystem zur Steuerung einer der oben dargestellten Vorrichtungen zur Bestrahlung ausgelegt; insbesondere auch in einer der bevorzugten Ausgestaltungen der Bestrahlungsvorrichtung.

Grundsätzlich betrifft die Erfindung auch ein entsprechendes vorteilhaftes Verfahren zum Erzeugen eines Datensatzes, insbesondere auf der Basis des Planungsverfahrens, ein Verfahren zum Steuern einer Bestrahlungsvorrichtung, insbesondere auf der Basis des Bestrahlungsverfahrens und ein entsprechendes vorteilhaftes Computer-Programmprodukt, auf der Basis der Erfindung.

Vorzugsweise werden bestimmte Dosisbeiträge, etwa quantifiziert als Partikelzahlen, zu einem Datensatz zusammengefasst, der insbesondere zum Steuern einer Bestrahlungsvorrichtung in einem kontinuierlichen oder diskontinuierlichen Prozess geeignet ist.

Dieser Datensatz kann als Steuerdatensatz verwendet werden, der zum Steuern einer Vorrichtung zum Bestrahlen während des Applizierens etwa einer Fraktion verwendbar ist. Der Datensatz kann darüber hinaus die Koordinaten bzw. x- und y-Positionen und die Partikelenergien oder die entsprechenden z-Positionen der Zielpunkte definieren. Diese Koordinaten können auf der Grundlage aktueller Daten jeweils angepasst werden.

Der Dosisbeitragsdatensatz für die einzelnen Zielpunkte in verschiedenen Tiefen kann generisch vorliegen, so dass nicht jedes Mal eine Berechnung erfolgen muss und außerdem Dosisbeiträge aller Zielpunkte mit dem generischen Ansatz beschrieben werden können.

Die vorangehende und die folgende Beschreibung der einzelnen Merkmale bezieht sich sowohl auf sämtliche Gegenstände nach der Verfahrenskategorie als auch nach der Vorrichtungskategorie, ohne dass dies im Einzelnen in jedem Fall explizit erwähnt ist; die dabei offenbarten Einzelmerkmale können auch in anderen als den gezeigten Kombinationen erfindungswesentlich sein.

Die vorangehende und die folgende Beschreibung der einzelnen Merkmale bezieht sich weiter auch auf ein Computerprogramm-Produkt mit Programmcode zur Durchführung erfindungsgemäßer Verfahren und/oder zur Implementierung auf einem Rechner/Prozessor.

Ferner ist die Erfindung als Computerprogramm-Produkt mit auf einem maschinenlesbaren Träger, beispielsweise einem ROM-, EPROM-, EEPROM- oder Flash-Speicher, einer CD-ROM oder DVD oder auf einer Diskette oder Festplatte, oder in Form von Firmware gespeicherten Programmcode zur Durchführung von einem der genannten Verfahren, wenn das Computerprogramm-Produkt auf einem Computer, Rechner oder Prozessor abläuft, implementierbar.

Ferner kann die vorliegende Erfindung als digitales Speichermedium, beispielsweise ROM-, EPROM-, EEPROM- oder Flash-Speicher, CD-ROM oder DVD oder Diskette oder Festplatte, mit elektronisch auslesbaren Steuersignalen, die so mit einem programmierbaren Computer- oder Prozessor-System zusammenwirken können, dass eines der beschriebenen Verfahren ausgeführt wird, implementiert werden.

Im Folgenden soll die Erfindung auch anhand von Ausführungsbeispielen näher erläutert werden.

Nachfolgend werden Ausführungsbeispiele mit Bezug auf die beiliegenden Figuren näher erläutert. Es zeigen:
- Fig. 1: eine schematische Darstellung einer Bestrahlungsvorrichtung;
- Fig. 2: eine schematische Darstellung von Einrichtungen zur Bestrahlung;
- Fig. 3: drei schematische Darstellungen von zwei Zielvolumen in einem Schnitt senkrecht zur Strahlrichtung;
- Fig. 4: zwei schematische Darstellungen von zwei Zielvolumen in einem Schnitt parallel zur Strahlrichtung;
- Fig. 5: ein Ablaufschema des Verfahrens;
- Fig. 6: schematische Aufzählung ausgestaltender Verfahrensschritte.

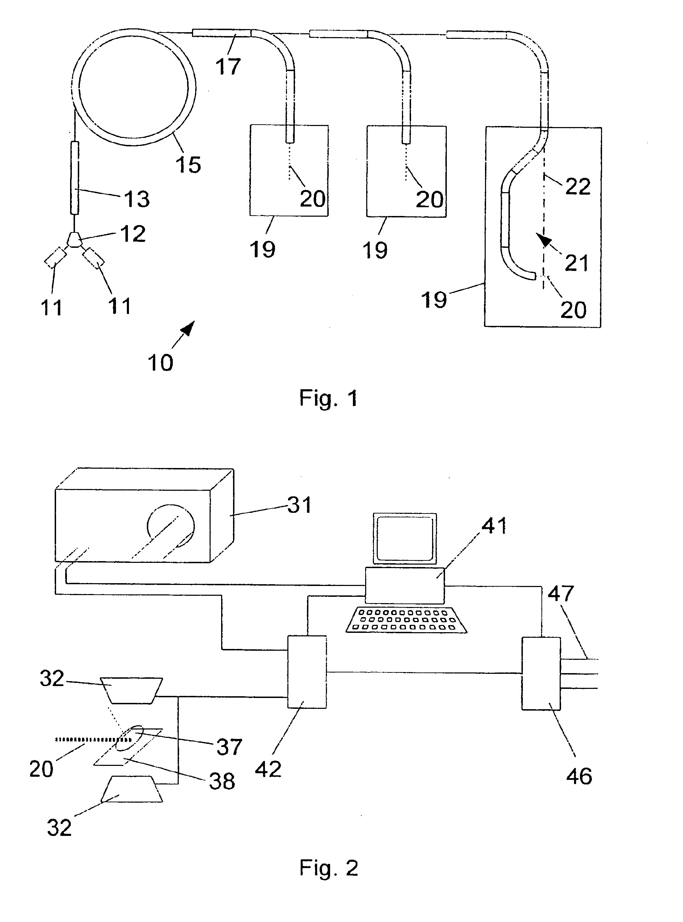

Figur 1 zeigt einen schematischen Überblick über den Aufbau einer Bestrahlungsvorrichtung 10 als Beispiel für eine Anlage zum Bestrahlen eines Materials bzw. Körpers, insbesondere eines tumorerkrankten Gewebes in dem Körper, mit einem Partikelstrahl. Als Partikel werden vornehmlich Ionen, beispielsweise Protonen, Pionen, Heliumionen, Kohlenstoffionen, Neonionen etc., eingesetzt. Grundsätzlich kann die Erfindung auch mit einer Photonenquelle ausgeführt werden.

Üblicherweise werden derartige Partikel in einer Partikelquelle 11 erzeugt. Die dargestellte Anlage 10 verfügt über zwei Partikelquellen 11, die zwei verschiedene Ionensorten erzeugen; hier kann zwischen diesen beiden Ionensorten mit einem Schaltmagnet 12 umgeschaltet werden.

Die Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Ionen in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Ionen den Beschleuniger 15 verlassen, führt ein Hochenergiestrahl-Transportsystem 17 den Partikelstrahl 20 zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus, vgl. die beiden weiter links der drei dargestellten Räume 19, oder aber über eine um eine Achse 22 bewegliche rotierbare Gantry 21 von verschiedenen Richtungen aus.

Figur 2 zeigt eine schematische Darstellung von Einrichtungen, die für eine Bestrahlung nach der Erfindung verwendet werden können.

Mittels eines Computer-Tomographen oder Kernspin-Tomographen 31 oder mittels anderer diagnostischer Einrichtungen können Lage und Ausdehnung eines zu bestrahlenden Tumors oder eines anderen Zielvolumens ermittelt werden. Daten von dem Tomographen 31 werden unmittelbar oder nach einer Aufbereitung durch weitere, in Figur 2 nicht dargestellte Einrichtungen, in einer Vorrichtung 41 zum Erstellen eines Datensatzes verarbeitet. Die Vorrichtung 41 ist etwa ein Arbeitsplatz-Computer, eine Workstation oder ein anderer Rechner. Optional ist die Vorrichtung 41 ferner durch ihre Benutzerschnittstelle, Software oder andere Merkmale dafür geeignet, dass medizinisches Personal dort die Zielvolumen, die zu applizierenden Dosen, die Aufteilung derselben auf mehrere Durchgänge, die Richtung der Bestrahlung und andere Details der Bestrahlung definiert.

Die Bestrahlungsvorrichtung wird mit der Vorrichtung 41 und auch mit Steuereinrichtungen 42, 46 sowie ggf. zusätzlich auch über eine Steuerleitung 47 gesteuert. Die Steuereinrichtungen 42 und 46 enthalten Einrichtungen zur Bestimmung des Status der Bestrahlungsvorrichtung 10, etwa Scannereinstellung und Strahleigenschaften, sowie Einrichtungen zum Einstellen der Bestrahlungsvorrichtung 10, etwa eine Scannersteuerung. Vorrichtung 41 und Steuereinrichtungen 42 und 46 entsprechen zusammen dem Kontrollsystem der Bestrahlungsvorrichtung 10.

Ein zu bestrahlender Körper 37 kann mit verschieden ausgebildeten Überwachungseinrichtungen vor, während oder nach der Bestrahlung durch die Anlage 10 überwacht werden. Beispielsweise sind eine PET-Kamera 32 (PET = Positronen-Emissions-Tomographie) und/oder ein in Figur 2 nicht dargestellter Computer-Tomograph zur Erfassung eines zu bestrahlenden Körpers 37, der auf einer Lagerfläche 38 gelagert ist, vorgesehen. Die PET-Kamera 32 und die Lagerfläche 38 können innerhalb eines der oben anhand der Figur 1 dargestellten Bestrahlungsräume 19 angeordnet sein. In diesem Fall können mittels der PET-Kamera 32 und/oder Computer-Tomographen die durch den Partikelstrahl 20 erzeugte Dosis sowie Position, Größe und Form der Zielvolumen in dem zu bestrahlenden Körper 37 erfasst werden. Alternativ sind die PET-Kamera 32 und die Lagerfläche 38 außerhalb eines Bestrahlungsraums angeordnet. Alternativ oder zusätzlich kann eine Tomographie des Körpers 37 mittels einer Fluoroskopie-Einrichtung, einer Röntgen-Einrichtung, eines Ultraschallsensors und/oder anderer ein dreidimensionales Bild gebender Einrichtungen erstellt werden. Die Bildgebung kann direkt im Bestrahlungsraum erfolgen, jedoch auch außerhalb - bevor der Patient in den Bestrahlungsraum gebracht wird.

Der anhand der Figur 1 dargestellte Grundaufbau einer Bestrahlungsanlage 10 ist typisch für viele Partikeltherapieanlagen und andere Bestrahlungsanlagen, ein abweichender Aufbau ist aber ebenfalls möglich. Die nachfolgend beschriebenen Ausführungsbeispiele sind sowohl in Zusammenhang mit der anhand der Figur 1 dargestellten Bestrahlungsanlage und den oben anhand der Figur 2 dargestellten Einrichtungen als auch mit anderen Bestrahlungsanlagen und Einrichtungen einsetzbare.

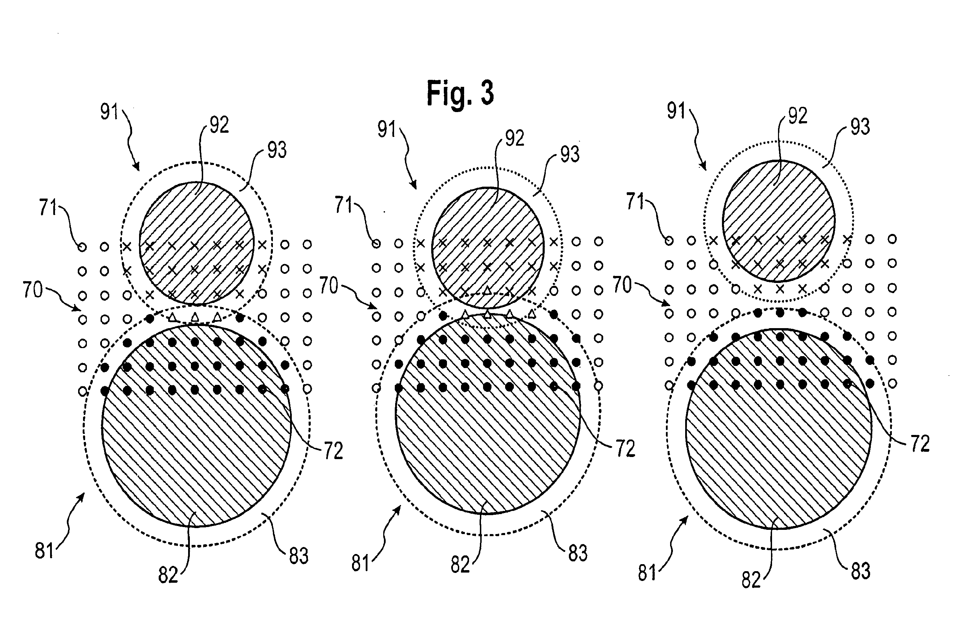

Die Figur 3 zeigt einen zur Richtung des Partikelstrahls senkrechten Schnitt innerhalb einer Isoenergieschicht bei drei verschiedenen relativen räumlichen Anordnungen von zwei Zielvolumen 81 und 91.

Unten ist jeweils ein Planungszielvolumen 81 mit einem inneren Bereich 82, schraffiert, und einem Sicherheitssaum 83, mit einer gepunktete Linie eingefasst, gezeigt. Oben ist jeweils ein kleineres Planungszielvolumen 91 mit einem innneren Bereich 92, schraffiert, und einem Sicherheitssaum 93, mit einer gepunktete Linie eingefasst, gezeigt.

In der linken Darstellung überlappen sich die Zielvolumen 81 und 91; in der mittleren Darstellung ist der Überlapp etwas größer und in der rechten Darstellung ist kein Überlapp gegeben.

Leere Kreise stehen in der Figur 3 für Rasterpunkte 71, die nicht angefahren werden. Die Rasterpunkte 71 können etwa in kubischen, rechtwinkligen, hexagonalen oder anderen Rastern 70 angeordnet sein. Rasterpunkte 71 innerhalb der Zielvolumen 81 und 91 werden hingegen angefahren. In diesem Beispiel fallen die Positionen der Zielpunkte 72 mit Rasterpunkten 71 zusammen. Die innerhalb des Zielvolumens 81 liegenden Zielpunkte 72 sind dem Volumen 81 zugeordnet. Die innerhalb des Zielvolumens 91 liegenden Zielpunkte 72 sind dem Volumen 91 zugeordnet. Da die Zielvolumen 81 und 91 überlappen, fällt ein Teil der Zielpunkte in diesen Überlapp. Hier ergibt sich die Situation, dass auf diese Rasterpunkte sowohl ein dem Zielvolumen 81 zugeordneter Zielpunkt als auch ein dem Zielvolumen 91 zugeordneter Zielpunkt fällt. Diese Positionen sind durch leere Dreiecke dargestellt. Die ausschließlich dem Zielvolumen 81 zugeordneten Zielpunkte sind durch volle Kreise dargestellt und die ausschließlich dem Zielvolumen 91 zugeordneten Zielpunkte sind durch Kreuze dargestellt.

Die Zieldosisverteilungen für die beiden Zielvolumen 81 und 91 werden unabhängig voneinander geplant. Aufgrund des Überlapps würde sich ohne eine Anpassung hier innerhalb des Überlapps eine Überdosierung ergeben.

Der Überlapp wird jedoch im Vorfeld oder auch während einer Bestrahlung, etwa zwischen den Durchgängen, festgestellt; es kann erfindungsgemäß angepasst werden.

Soll beispielsweise das größere Zielvolumen 81 homogen mit einer Zieldosis A und dass kleinere Zielvolumen 91 homogen mit einer Zieldosis B belegt werden, so können die mit einem Kreuz markierten Rasterpunkte entsprechend der Zieldosis B und die mit einem vollen Kreis markierten Rasterpunkte entsprechend der Zieldosis A angefahren werden. Sei hier die Dosis A etwa größer als die Dosis B. Gewünscht sei es, die Anpassung so vorzunehmen, dass auch innerhalb des Überlapps die Dosis A deponiert wird. Dazu werden die in den Überlapp fallenden dem oberen Zielvolumen 91 zugeordneten Zielpunkte als inaktiv gekennzeichnet und die in den Überlapp fallenden dem unteren Zielvolumen 81 zugeordneten Zielpunkte als aktiv gekennzeichnet. Insgesamt werden dann nach dem entsprechend angepassten Plan die in dem Überlapp liegenden Rasterpunkte zur Erzielung der Dosis A entsprechend angefahren.

Die mittlere Darstellung in Fig. 3 zeigt den Fall, dass die Zielvolumen 81 und 91 weiter aufeinander zu gerückt sind. Der Überlapp ist entsprechend größer. Man erkennt etwa, dass ein in der linken Darstellung ausschließlich in dem oberen Zielvolumen 91 liegender Rasterpunkt nun in den Überlapp fällt (oberste Zeile innerhalb des Überlapps). Hier liegt ein nun inaktiver dem oberen Zielvolumen 91 zugeordneter Zielpunkt und ein aktiver dem unteren Zielvolumen 81 zugeordneter Zielpunkt. Insgesamt wird nun auch hier die Dosis A appliziert.

Die rechte Darstellung in Fig. 3 zeigt den Fall, dass die Zielvolumen 81 und 91 weiter voneinander weg gerückt sind. Hier ist kein Überlapp mehr gegeben. Eine Intensitätsanpassung ist nicht erforderlich. Sämtliche Zielpunkte sind als aktiv gekennzeichnet. Es wird jedoch weiter von Durchgang zu Durchgang geprüft, ob sich nicht vielleicht doch eine Überlagerung ergibt.

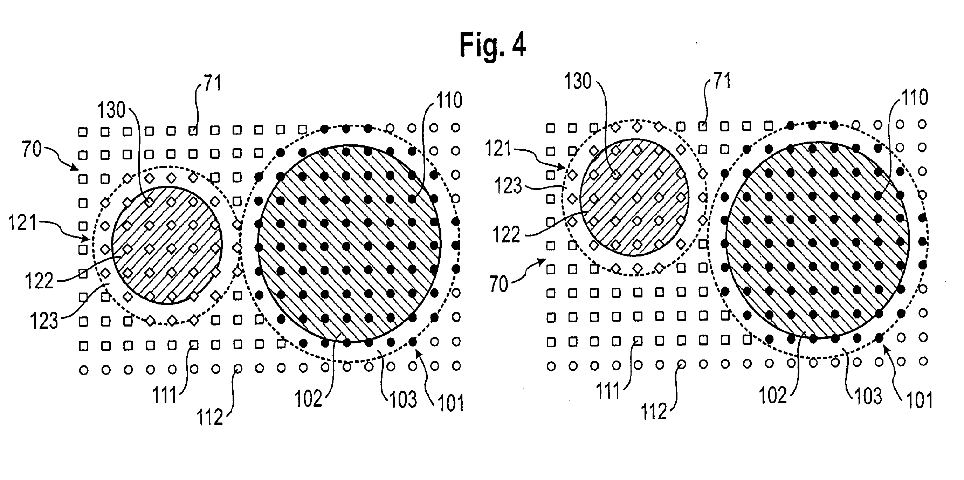

Die Figur 4 zeigt zwei Schnitte parallel zur Richtung eines von links einfallenden Partikelstrahls. In der linken Darstellung ist rechts ein größeres Zielvolumen 101 dargestellt. Dieses umfasst einen inneren Bereich 102 und einen Sicherheitssaum 103. Innerhalb des Zielvolumens 101 befinden sich auf einem Raster 70 mit Rasterpunkten 71 liegende Zielpunkte 110, volle Kreise, die mit Rasterpunkten 71 zusammenfallen, und dem rechten Zielvolumen 101 zugeordnet sind.

In dem auf das rechte Zielvolumen 101 von links zulaufenden Einstrahlungskanal ist ebenfalls eine Dosis deponiert. Die entsprechenden Rasterpunkte 71 sind mit leeren Vierecken markiert. Diese Punkte können ebenfalls dem rechten Zielvolumen 101 zugeordnet werden; dies ist jedoch nicht obligatorisch. Es bietet sich an, zu diesen Punkten auch die Information zu speichern, dass sie außerhalb des Zielvolumens 101 liegen. Diese Punkte können aber auch mit einem Index gekennzeichnet werden, welcher sich von der Zuordnung von Zielpunkten zu Zielvolumen unterscheidet und der ausdrückt, dass diese Punkte mit dem rechten Zielvolumen 101 assoziiert sind, jedoch nicht innerhalb dieses Zielvolumens 101 liegen bzw. keine Zielpunkte des Zielvolumens 101 sind. Alternativ kann zu diesen Punkten auch die Information gespeichert werden, dass hier eine Dosis deponiert ist und diese Dosis beim Bestrahlen des rechten Zielvolumens 101 entstand.

In dem Einstrahlungskanal liegt ein kleineres Planungszielvolumen 121, mit einem inneren Bereich 122 und einem Sicherheitssaum 123.

Ein Anfahren von in dem kleineren Zielvolumen 121 liegenden und diesem zugeordneten Zielpunkten 130 führt zu einer Überlagerung mit der durch das Anfahren der Zielpunkte 110 verursachten Deposition. Sind die Zieldosisverteilungen für die Zielvolumen 101 und 121 unabhängig voneinander geplant und erfolgt keine Anpassung, so übersteigt die in dem linken Zielvolumen 121 deponierte Dosis die gewünschte Zieldosis.

Hier aber wird die durch die Bestrahlung des rechten Zielvolumens 101 verursachte Dosis an den mit den Rauten gekennzeichneten Rasterpunkten berücksichtigt. Die Anpassung besteht darin, dass die dem linken Zielvolumen 121 zugeordneten Zielpunkte 130 mit einem entsprechend weniger intensiven Strahl angefahren werden als es der Zieldosis des Zielvolumens 121 alleine entspricht.

Ergeben sich von einem Durchgang zum nächsten Durchgang relativ zueinander verschobene Zielvolumen, so sollten bei der Anpassung für das linke Zielvolumen 121 andere Dosisbeiträge, berücksichtigt werden, vgl. rechte Darstellung in Fig. 4, Rauten.

Die in den Abbildungen 3 und 4 dargestellten Fälle, also Überlapp von Zielvolumen und Überlagerung mit außerhalb eines Zielvolumens deponierter Dosis, sind der Einfachheit halber getrennt dargestellt. Nach der Erfindung können diese Fälle auch in Kombination vorliegen.

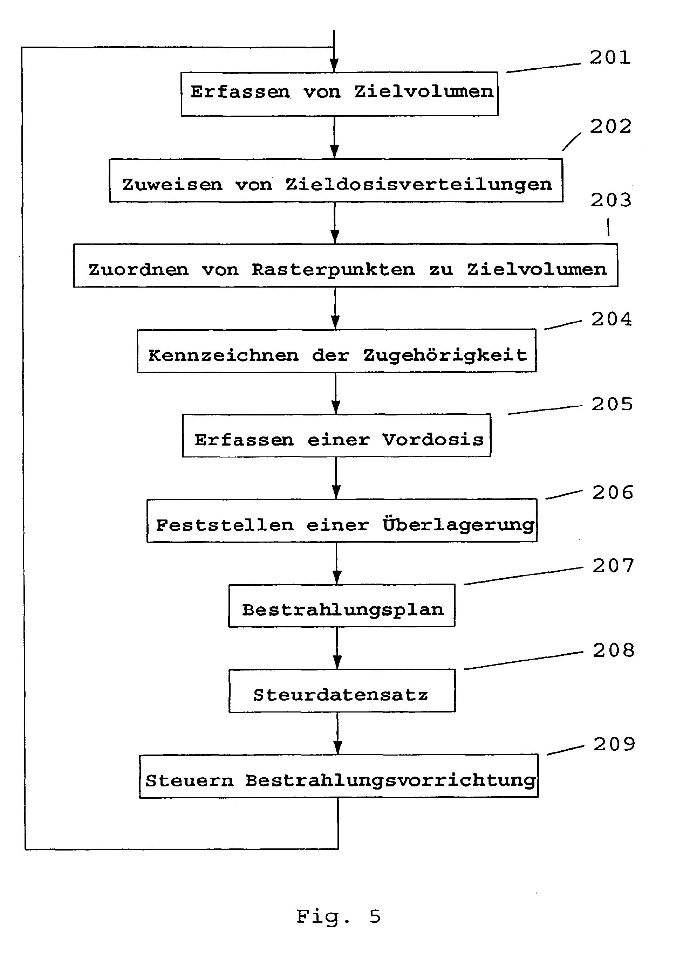

In Fig. 5 ist schematisch ein möglicher Verfahrensablauf dargestellt.

In einem ersten Schritt 201 werden die Zielvolumen nach einer der oben vorgeschlagenen Methoden erfasst. In einem zweiten Schritt 202 werden diesen Zielvolumen Zieldosisverteilungen zugewiesen. Lage und Zieldosisverteilungen können schon jetzt etwa in einen später zur Steuerung der Bestrahlungsvorrichtung geeigneten Steuerdatensatz geschrieben werden; beispielsweise pro Volumenelement des Ziels oder pro Rasterpunkt.

In einem dritten Schritt 203 werden die Rasterpunkte den Zielvolumen zugeordnet und deren Zugehörigkeit zu einem der Zielvolumen in einem vierten Schritt 204 gekennzeichnet. Die Kennzeichnung kann auch in den Steuerdatensatz mit aufgenommen werden.

In einem fünften Schritt 205 werden Vordosen aus vorangegangenen Durchgängen erfasst. Dies erfolgt beispielsweise durch eine numerische Simulation der zurückliegenden Durchgänge aufgrund der dabei jeweils tatsächlich vorliegenden Situationen und gemessenen Einstellungen der Bestrahlungsvorrichtung. Alternativ wird die in jedem Durchgang applizierte Dosis bereits während der Bestrahlung durch Positronen-Emissions-Tomographie von bei der Bestrahlung erzeugten Nukliden, die Positronen emittieren, erfasst. Alternativ kann während der Bestrahlung nach jedem Durchgang auf Basis der gemessenen Bestrahlungsparameter eine Simulation erfolgen deren Ergebnisse im folgenden Durchgang berücksichtigt werden.

Weiter werden in einem sechsten 206 Schritt Überlagerungen festgestellt.

Auf Basis der erfassten Vordosen und der festgestellten Überlagerungen wird in einem siebten Schritt 207 der Bestrahlungsplan erstellt bzw. angepasst. Dabei werden insbesondere die Partikelzahlen auf der Grundlage der erfassten gegenwärtigen Situation, insbesondere betreffend die Positionen, Größen und Formen der Zielvolumen bestimmt. Anstelle der Partikelzahl können andere äquivalente Größen bestimmt werden, beispielsweise die elektrostatische Gesamtladung der auf den Zielpunkt gerichteten Partikel.

Spätestens jetzt wird in einem achten Schritt der Steuerdatensatz erstellt, ansonsten angepasst. In einem neunten Schritt wird er zum Steuern der Bestrahlungsvorrichtung benutzt.

Die Schritte 201 bis 209 können etwa vor jedem Durchgang wiederholt werden. Auch eine Iteration während eines Durchgangs ist grundsätzlich nicht ausgeschlossen.

Ohne Überlagerung/Überlapp können alle Rasterpunkte als aktiv gekennzeichnet verbleiben.

Die jeweils zu einem Zielvolumen gehörenden Rasterpunkte können durch geeignete Transformationen, die für unterschiedliche Zielvolumen verschieden sein können, angepasst werden. So lassen sich auch verschiedene interne Bewegungen der Zielvolumen berücksichtigen, was etwa bei einer reinen Positionskorrektur, etwa eines Patienten, in der Regel nicht möglich ist.

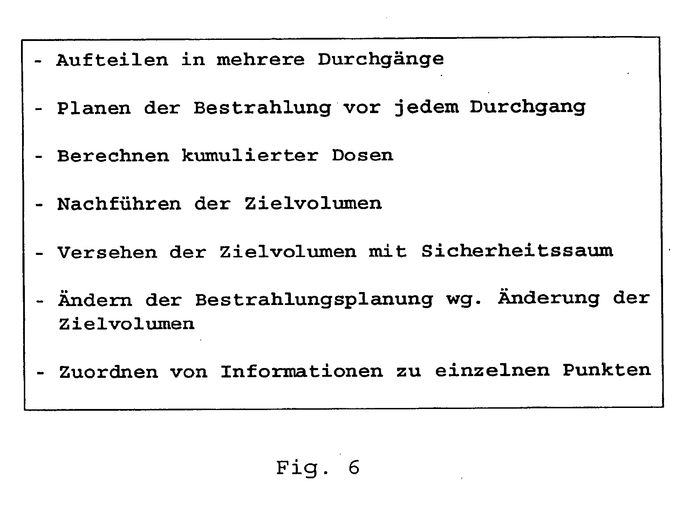

Fig. 6 zeigt eine Aufzählung ausgestaltender Verfahrensschritte darunter:
- Aufteilen der Bestrahlung in mehrere Durchgänge, so dass in jedem Durchgang ein Teil der Zieldosis appliziert wird, sich in der Summe jedoch die Gesamtdosis ergibt;
- Planen der Bestrahlung vor jedem Durchgang;
- Nachführen der Zielvolumen bzw. entsprechendes Anpassen der einzelnen Rasterpunkte;
- Versehen der Zielvolumen mit jeweils einem Sicherheitssaum;
- Ändern der Bestrahlungsplanung basierend auf einer Änderung der Form und/oder Lage der Zielvolumen;
- Zuordnen von Information zu den Zielpunkten des zu bestrahlenden Körpers; hinausgehend über eine bloße Zuordnung zu einem Zielvolumen.

Im Rahmen einer erfindungsgemäßen adaptiven Bestrahlung, insbesondere mit Partikeln, mit gescanntem Strahl können auch Bestrahlungspläne berücksichtigt werden, in die mehrere Zielvolumen einfließen; vor allem auch bei angrenzenden bzw. sich überlappenden Zielvolumen. Ohne diese Möglichkeit wären bei nicht überlappenden Zielvolumen Sicherheitssäume zu berücksichtigen, die die Mobilität aller Zielvolumen beinhalten. Das heißt, wenn sich ein Zielvolumen entlang der Körperhauptachse bewegt und ein anderes senkrecht dazu, müssen für beide Zielvolumen Sicherheitssäume in beiden Richtungen berücksichtigt werden, wenn die Bestrahlung nicht individuell den Zielvolumen angepasst werden kann.

In der Regel ist einer Bestrahlung der Zielvolumen getrennt voneinander mit jeweils einzelner Adaption wohl nicht wünschenswert, wenn die Zielvolumen überlappen. Dies führt nämlich bei einzelner Anwendung der Felder zu einer Überdosierung im Überlappbereich, da von beiden Feldern jeweils die dafür vorgeschriebene Dosis appliziert wird. In solchen Fällen ist die Erfindung Voraussetzung dafür, dass eine konforme Bestrahlung durchgeführt werden kann.

Die beschriebenen Ausführungsbeispiele sind nicht nur für eine Anwendung im Rahmen einer Partikeltherapie geeignet. Sie sind darüber hinaus allgemein in Vorrichtungen zur Bestrahlung von Materie anwendbar, unabhängig davon, ob es sich um lebende oder tote, organische oder anorganische Substanz, einen Körper eines Patienten oder eines zu behandelnden Tieres, ein Phantom, eine Materialprobe oder eine Vorrichtung handelt.

### Bezugszeichenliste

- 10: Bestrahlungsvorrichtung
- 11: Partikelquelle
- 12: Schaltmagnet
- 13: Vorbeschleuniger
- 15: Beschleuniger
- 17: Hochenergiestrahl-Transportsystem
- 19: Bestrahlungsraum
- 20: Partikelstrahl
- 21: Gantry
- 22: Achse der Gantry 21
- 31: Tomograph
- 32: PET-Kamera
- 37: Körper
- 38: Lagerfläche
- 41: Rechner
- 42: Steuereinrichtung
- 46: Steuereinrichtung
- 47: Steuerleitung
- 70: Raster
- 71: Rasterpunkt
- 72: Zielpunkt
- 81: Zielvolumen
- 82: innerer Bereich
- 83: Sicherheitssaum
- 91: Zielvolumen
- 92: innerer Bereich
- 93: Sicherheitssaum
- 101: Zielvolumen
- 102: innerer Bereich
- 103: Sicherheitssaum
- 110: Zielpunkt
- 121: Zielvolumen
- 122: innerer Bereich
- 123: Sicherheitssaum

- 201: erster Schritt
- 202: zweiter Schritt
- 203: dritter Schritt
- 204: vierter Schritt
- 205: fünfter Schritt
- 206: sechster Schritt
- 207: siebter Schritt
- 208: achter Schritt
- 209: neunter Schritt

## Patentansprüche

1. Verfahren zur Bestrahlung von zwei in einem Körper angeordneten Zielvolumen (81, 91, 101, 121), die nicht in dem Körper eines Patienten oder Tieres vorliegen, mit einem Zielpunkte (72, 110) anfahrenden Strahl (20) zur Deposition einer ersten Zieldosisverteilung in einem ersten der zwei zielvolumen und einer zweiten Zieldosisverteilung in einem zweiten der
zwei Zielvolumen, **gekennzeichnet durch** die Schritte:
Zuordnen von Zielpunkten (72, 110) zu einem der zielvolumen (81, 91, 101, 121),
Feststellen einer Überlagerung von einer ersten Deposition verursacht **durch** das Anfahren eines dem ersten Zielvolumen, (81, 91, 101, 121) zugeordneten Zielpunkts (72, 110) mit einer zweiten Deposition verursacht **durch** das Anfahren eines dem zweitem Zielvolumen (81, 91, 101, 121) zugeordneten Zielpunkts (72, 110),
Anpassen der Bestrahlung für zumindest einen der Zielpunkte (72, 110), dessen Anfahren zu der Überlagerung der ersten und zweiten Deposition beiträgt, mittels zumindest einem der Schritte:
- Inaktivieren des in der Überlagerung liegenden Zielpunkts in einem der Zielvolumen, wobei der Zielpunkt für die geringere Zieldosisverteilung inaktiviert wird und der Zielpunkt für die höhere Zieldosisverteilung aktiv bleibt, so dass im Überlapp nur noch die Zielpunkte des Zielvolumens entsprechend der höheren Zieldosisverteilung angefahren werden,
- Verringerung der deponierten Dosis für den in der Überlagerung liegenden Zielpunkt
und
Erfassen der Zielvolumen mittels eines zur dreidimensionalen Abbildung, oder auch 2D oder 4D Abbildung, des Körpers geeigneten Verfahrens, um Veränderungen von Position, Größe und Form gegenüber der Situation, die einem ursprünglichen Bestrahlungsplan zugrunde lag, zu erfassen.

2. Vorrichtung (10) zur Bestrahlung von zwei in einem Körper angeordneten Zielvolumen (81, 91, 101, 121) mit einem Zielpunkte (72, 110) anfahrenden Strahl (20) zur Deposition einer ersten Zieldosisverteilung in einem ersten der zwei Zielvolumen (81, 91, 101, 121) und einer zweiten Zieldosisverteilung in einem zweiten der zwei Zielvolumen (81, 91, 101, 121) mit einer Strahlenquelle (11) und
einem Kontrollsystem (41, 42, 46, 47) zur Steuerung der Vorrichtung (10),
**dadurch gekennzeichnet, dass** das Kontrollsystem (41, 42, 46, 47) dazu ausgelegt ist,
Zielpunkte (72, 110) zu einem der Zielvolumen (81, 91, 101, 121) zuzuordnen,
eine Überlagerung von einer ersten Deposition verursacht durch das Anfahren eines dem ersten zielvolumen (81, 91, 101, 121) zugeordneten Zielpunkts (72, 110) mit einer zweiten Deposition verursacht durch das Anfahren eines dem zweiten Zielvolumen (81, 91, 101, 121) zugeordneten Zielpunkts (72, 110) festzustellen,
die Bestrahlung für zumindest einen der Zielpunkte (72, 110), dessen Anfahren zu der Überlagerung der ersten und zweiten Deposition beiträgt, mittels zumindest einem der Schritte:
- Inaktivieren des in der Überlagerung liegenden Zielpunkts in einem der Zielvolumen, wobei der Zielpunkt für die geringere Zieldosisverteilung inaktiviert wird und der Zielpunkte für die höhere zieldosisverteilung aktiv bleibt, so dass im Überlapp nur noch die Zielpunkte des Zielvolumens entsprechend der höheren Zieldosisverteilung angefahren werden,
- Verringerung der deponierten Dosis für den in der Überlagerung liegenden Zielpunkt
anzupassen und
die zielvolumen mittels eines zur dreidimensionalen Abbildung, oder auch 2D oder 4D Abbildung, des Körpers geeigneten Verfahrens zu erfassen, um Veränderungen von Position, Größe und Form gegenüber der Situation, die einem ursprünglichen Bestrahlungsplan zugrunde lag, zu erfassen.

3. Vorrichtung (10) nach Anspruch 2, ausgelegt zur Durchführung eines Verfahrens zur Bestrahlung nach Anspruch 1.

4. Kontrollsystem (41, 42, 46, 47) zur Steuerung einer Vorrichtung (10) zur Bestrahlung von zwei in einem Körper angeordneten Zielvolumen (81, 91, 101, 121) mit einem Zielpunkte (72, 110) anfahrenden Strahl (20) zur Deposition einer ersten Zieldosisverteilung in einem ersten der zwei Zielvolumen (81, 91, 101, 121) und einer zweiten Zieldosisverteilung in einem zweiten der zwei zielvolumen (81, 91, 101, 121),
**dadurch gekennzeichnet, dass** das Kontrollsystem (41, 42, 46, 47) dazu ausgelegt ist, ziel punkte (72, 110) zu einem der zielvolumen (81, 91, 101, 121) zuzuordnen,
eine Überlagerung von einer ersten Deposition verursacht durch das Anfahren eines dem ersten Zielvolumen (81, 91, 101, 121) zugeordneten Zielpunkts (72, 110) mit einer zweiten Deposition verursacht durch das Anfahren eines dem zweiten Zielvolumen (81, 91, 101, 121) zugeordneten Zielpunkts (72, 110) festzustellen,
die Bestrahlung für zumindest einen der Zielpunkte (72, 110), dessen Anfahren zu der Überlagerung der ersten und zweiten Deposition beiträgt, mittels zumindest einem der Schritte:
- Inaktivieren des in der Überlagerung liegenden Zielpunkts in einem der Zielvolumen, wobei der Zielpunkt für die geringere Zieldosisverteilung inaktiviert wird und der Zielpunkt für die höhere Zieldosisverteilung aktiv bleibt, so dass im Überlapp nur noch die Zielpunkte des zielvolumens entsprechend der höheren Zieldosisverteilung angefahren werden,
- Verringerung der deponierten Dosis für den in der Überlagerung liegenden Zielpunkt
anzupassen, und
die Zielvolumen mittels eines zur dreidimensionalen Abbildung, oder auch 2D oder 4D Abbildung, des Körpers geeigneten Verfahrens zu erfassen, um Veränderungen von Position, Größe und Form gegenüber der Situation, die einem ursprünglichen Bestrahlungsplan zugrunde lag, zu erfassen.

5. KontrollSystem (41, 42, 46, 47) nach Anspruch 4 zur Steuerung einer Vorrichtung (10) nach einem der Ansprüche 2 oder 3.

## Claims

1. Method for irradiating two target volumes (81, 91, 101, 121) disposed in a body, which target volumes are not present in the body of a patient or an animal, with a beam (20) moving to target points (72, 110), for depositing a first target dose distribution in a first one of the two target volumes and a second target dose distribution in a second one of the two target volumes, **characterized by** the following steps:
assigning target points (72, 110) to one of the target volumes (81, 91, 101, 121),
determining a superposition of a first deposition caused by moving to a target point (72, 110) assigned to the first target volume (81, 91, 101, 121) with a second deposition caused by moving to a target point (72, 110) assigned to the second target volume (81, 91, 101, 121),
modifying the irradiation on at least one of the target points (72, 110), the movement to which contributes to the superposition of the first and
second deposition, by means of at least one of the following steps:
- inactivating the target point situated in the superposition in one of the target volumes, wherein the target point is inactivated for the lower target dose distribution and the target point for the higher target dose distribution remains active, such that only the target points of the target volume corresponding to the higher target dose distribution are approached in the overlap,
- reducing the deposited dose for the target point lying in the overlap
and
acquiring the target volumes by means of a method suitable for three-dimensional imaging, or else 2D or
4D imaging, of the body in order to acquire changes in position, size and form with respect to the situation on which an original radiation plan was based.

2. Device (10) for irradiating two target volumes (81, 91, 101, 121) disposed in a body, comprising a beam (20) moving to target points (72, 110), for depositing a first target dose distribution in a first one of the two target volumes (81, 91, 101, 121) and a second target dose distribution in a second one of the two target volumes (81, 91, 101, 121), comprising a radiation source (11) and a control system (41, 42, 46, 47) for controlling the device (10), **characterized in that** the control system (41, 42, 46, 47) is configured
to assign target points (72, 110) to one of the target volumes (81, 91, 101, 121),
to determine a superposition of a first deposition caused by the movement to a target point (72, 110) assigned to the first target volume (81, 91, 101, 121) with a second deposition caused by the movement to a target point (72, 110) assigned to the second target volume (81, 91, 101, 121),
to modify the irradiation on at least one of the target points (72, 110), the movement to which contributes to the superposition of the first and second deposition, by means of at least one of the following steps:
- inactivating the target point situated in the superposition in one of the target volumes, wherein the target point is inactivated for the lower target dose distribution and the target point for the higher target dose distribution remains active, such that only the target points of the target volume corresponding to the higher target dose distribution are approached in the overlap,
- reducing the deposited dose for the target point lying in the overlap
and
to acquire the target volumes by means of a method suitable for three-dimensional imaging, or else 2D or 4D imaging, of the body in order to acquire changes in position, size and form with respect to the situation on which an original radiation plan was based.

3. Device (10) according to Claim 2, configured for carrying out a method for irradiating according to Claim 1.

4. Control system (41, 42, 46, 47) for controlling a device (10) for irradiating two target volumes (81, 91, 101, 121) disposed in a body, comprising a beam (20) moving to target points (72, 110), for depositing a first target dose distribution in a first one of the two target volumes (81, 91, 101, 121) and a second target dose distribution in a second one of the two target volumes (81, 91, 101, 121),
**characterized in that** the control system (41, 42, 46, 47) is configured
to assign target points (72, 110) to one of the target volumes (81, 91, 101, 121),
to determine a superposition of a first deposition caused by the movement to a target point (72, 110) assigned to the first target volume (81, 91, 101, 121) with a second deposition caused by the movement to a target point (72, 110) assigned to the second target volume (81, 91, 101, 121),
to modify the irradiation on at least one of the target points (72, 110), the movement to which contributes to the superposition of the first and second deposition, by means of at least one of the following steps:
- inactivating the target point situated in the superposition in one of the target volumes, wherein the target point is inactivated for the lower target dose distribution and the target point for the higher target dose distribution remains active, such that only the target points of the target volume corresponding to the higher target dose distribution are approached in the overlap,
- reducing the deposited dose for the target point lying in the overlap
and
to acquire the target volumes by means of a method suitable for three-dimensional imaging, or else 2D or 4D imaging, of the body in order to acquire changes in position, size and form with respect to the situation on which an original radiation plan was based.

5. Control system (41, 42, 46, 47) according to Claim 4 for controlling a device (10) according to either of Claims 2 and 3.

## Revendications

1. Procédé d'irradiation de deux volumes cibles (81, 91, 101, 121), disposés dans un corps, qui ne se trouvent pas dans le corps d'un patient ou d'un animal, avec un rayon (20) visant des points cibles (72, 110), en vue du dépôt d'une première distribution de dose cible dans un premier des deux volumes cibles, et d'une deuxième distribution de dose cible dans un deuxième des deux volumes cibles, **caractérisé en ce qu'**il comporte les étapes suivantes :
association de points cibles (72, 110) à l'un des volumes cibles (81, 91, 101, 121),
détermination d'une superposition d'un premier dépôt, provoqué par la visée d'un point cible (72, 110) associé au premier volume cible (81, 91, 101, 121), avec un deuxième dépôt, provoqué par la visée d'un point cible (72, 110) associé au deuxième volume cible (81, 91, 101, 121),
adaptation de l'irradiation pour au moins un des points cibles (72, 110) dont la visée contribue à la superposition du premier et du deuxième dépôt, au moyen d'au moins une des étapes suivantes :
- désactivation du point cible situé dans la superposition, dans l'un des volumes cibles, le point cible pour la distribution de dose cible plus faible étant désactivé et le point cible pour la distribution de dose cible plus élevée restant actif, de sorte que dans le chevauchement, seuls sont visés les points cibles du volume cible correspondant à la distribution de dose cible plus élevée,
- réduction de la dose déposée pour le point cible situé dans la superposition
et
détection des volumes cibles au moyen d'un procédé adapté à la représentation en trois dimensions, ou bien aussi la représentation en 2D ou 4D, du corps, pour détecter des changements de position, de taille et de forme par rapport à la situation sur laquelle se fondait un plan d'irradiation initial.

2. Dispositif (10) d'irradiation de deux volumes cibles (81, 91, 101, 121), disposés dans un corps, avec un rayon (20) visant des points cibles (72, 110), en vue du dépôt d'une première distribution de dose cible dans un premier des deux volumes cibles (81, 91, 101, 121), et d'une deuxième distribution de dose cible dans un deuxième des deux volumes cibles (81, 91, 101, 121), comprenant une source d'irradiation (11) et un système de contrôle (41, 42, 46, 47) pour commander le dispositif /10),
**caractérisé en ce que** le système de contrôle (41, 42, 46, 47) est conçu pour
associer des points cibles (72, 110) à l'un des volumes cibles (81, 91, 101, 121),
déterminer une superposition d'un premier dépôt, provoqué par la visée d'un point cible (72, 110) associé au premier volume cible (81, 91, 101, 121), avec un deuxième dépôt, provoqué par la visée d'un point cible (72, 110) associé au deuxième volume cible (81, 91, 101, 121),
adapter l'irradiation pour au moins un des points cibles (72, 110) dont la visée contribue à la superposition du premier et du deuxième dépôt, au moyen d'au moins une des étapes suivantes :
- désactivation du point cible situé dans la superposition, dans l'un des volumes cibles, le point cible pour la distribution de dose cible plus faible étant désactivé et le point cible pour la distribution de dose cible plus élevée restant actif, de sorte que dans le chevauchement, seuls sont visés les points cibles du volume cible correspondant à la distribution de dose cible plus élevée,
- réduction de la dose déposée pour le point cible situé dans la superposition,
et
détecter les volumes cibles au moyen d'un procédé adapté à la représentation en trois dimensions, ou bien aussi la représentation en 2D ou 4D, du corps, pour détecter des changements de position, de taille et de forme par rapport à la situation sur laquelle se fondait un plan d'irradiation initial.

3. Dispositif (10) selon la revendication 2, conçu pour la mise en oeuvre d'un procédé d'irradiation selon la revendication 1.

4. Système de contrôle (41, 42, 46, 47) pour commander un dispositif (10) d'irradiation de deux volumes cibles (81, 91, 101, 121), disposés dans un corps, avec un rayon (20) visant des points cibles (72, 110), en vue du dépôt d'une première distribution de dose cible dans un premier des deux volumes cibles (81, 91, 101, 121), et d'une deuxième distribution de dose cible dans un deuxième des deux volumes cibles (81, 91, 101, 121),
**caractérisé en ce que** le système de contrôle (41, 42, 46, 47) est conçu pour associer des points cibles (72, 110) à l'un des volumes cibles (81, 91, 101, 121),
déterminer une superposition d'un premier dépôt, provoqué par la visée d'un point cible (72, 110) associé au premier volume cible (81, 91, 101, 121), avec un deuxième dépôt, provoqué par la visée d'un point cible (72, 110) associé au deuxième volume cible (81, 91, 101, 121),
adapter l'irradiation pour au moins un des points cibles (72, 110) dont la visée contribue à la superposition du premier et du deuxième dépôt, au moyen d'au moins une des étapes suivantes :
- désactivation du point cible situé dans la superposition dans l'un des volumes cibles, le point cible pour la distribution de dose cible plus faible étant désactivé et le point cible pour la distribution de dose cible plus élevée restant actif, de sorte que dans le chevauchement, seuls sont visés les points cibles du volume cible correspondant à la distribution de dose cible plus élevée,
- réduction de la dose déposée pour le point cible situé dans la superposition,
et
détecter les volumes cibles au moyen d'un procédé adapté à la représentation en trois dimensions, ou bien aussi la représentation en 2D ou 4D, du corps, pour détecter des changements de position, de taille et de forme par rapport à la situation sur laquelle se fondait un plan d'irradiation initial.

5. Système de contrôle (41, 42, 46, 47) selon la revendication 4, destiné commander un dispositif (10) selon l'une des revendications 2 ou 3.
